# EUROPEAN PATENT SPECIFICATION

(11) **EP 1 575 608 B1**
(45) Date of publication and mention of the grant of the patent: **07.05.2008**
(21) Application number: 03812076.2
(22) Date of filing: 22.12.2003
(51) Int. Cl.: A61K 38/19, A61P 1/16

(54) **USE OF CC-CHEMOKINE CCL5/RANTES MUTANTS AGAINST LIVER DISEASES**
VERWENDUNG VON CC-CHEMOKINE CCL5/RANTES MUTANTEN ZUR BEHANDLUNG VON LEBERERKRANKUNGEN
UTILISATION DE MUTANTS DE LA CC-CHEMOKINE CCL5/RANTES POUR TRAITER DES MALADIES DU FOIE

(30) Priority: 23.12.2002 EP 02102885; 17.07.2003 EP 03077237
(43) Date of publication of application: 21.09.2005
(73) Proprietor: Laboratoires Serono SA, 1267 Coinsins, Vaud (CH)
(72) Inventor: PROUDFOOT, Amanda, F-74140 Chens sur Leman (FR); AJUEBOR, Maureen, Calgary, AB T2N 4K3 (CA); SWAIN, Mark, Calgary, AB T2T 3L9 (CA)
(74) Representative: Merck Serono International S.A. Intellectual Property
(86) International application number: PCT/EP2003/051090
(87) International publication number: WO 2004/062688

(56) References cited:
- EP-A- 1 325 751
- WO-A-02/28419
- WO-A-96/38559
- WO-A-98/13495
- WO-A-03/051921
- US-A- 5 739 103
- US-A- 5 965 697
- DATABASE WPI Section Ch, Week 200242 Derwent Publications Ltd., London, GB; Class B04, AN 2002-394437 XP002238282 & WO 02/30464 A (DAIICHI PHARM CO LTD) 18 April 2002 (2002-04-18)
- LAURENCE JENNIFER S ET AL: "Importance of basic residues and quaternary structure in the function of MIP-1beta: CCR5 binding and cell surface sugar interactions" BIOCHEMISTRY, AMERICAN CHEMICAL SOCIETY, EASTON, PA, US, vol. 40, no. 16, 24 April 2001 (2001-04-24), pages 4990-4999, XP002193384 ISSN: 0006-2960
- KOOPMANN WITTE ET AL: "Identification of a glycosaminoglycan-binding site in chemokine macrophage inflammatory protein-1-alpha" JOURNAL OF BIOLOGICAL CHEMISTRY, AMERICAN SOCIETY OF BIOLOGICAL CHEMISTS, BALTIMORE, MD, US, vol. 272, no. 15, 11 April 1997 (1997-04-11), pages 10103-10109, XP002171678 ISSN: 0021-9258
- FISHER N C ET AL: "Serum concentrations and peripheral secretion of the beta chemokines monocyte chemoattractant protein 1 and macrophage inflammatory protein 1alpha in alcoholic liver disease." GUT, vol. 45, no. 3, 1999, pages 416-420, XP009009464 ISSN: 0017-5749
- MIYAGUCHI S ET AL: "Elevated plasma RANTES in primary biliary cirrhosis patients" HEPATOLOGY RESEARCH 1997 IRELAND, vol. 8, no. 3, 1997, pages 156-163, XP009009465 ISSN: 0928-4346
- AJUEBOR MAUREEN ET AL: "Novel protective effects of the CC chemokine CCL2/MCP-1 in concanavalin A-induced acute hepatitis" GASTROENTEROLOGY, vol. 122, no. 4 Suppl. 1, April 2002 (2002-04), page A.637, XP009033937 & DIGESTIVE DISEASE WEEK AND THE 103RD ANNUAL MEETING OF THE AMERICAN GASTROENTEROLOGICAL ASSOCIATION; SAN FRANCISCO, CA, USA; MAY 19-22, 2002 ISSN: 0016-5085
- AJUEBOR MAUREEN ET AL: "The coordinated action of CC chemokines and their receptor may orchestrate hepatic inflammation during chronic experimental cholangitis." GASTROENTEROLOGY, vol. 123, no. 1 Supplement, July 2002 (2002-07), page 57, XP001194576 & DIGESTIVE DISEASE WEEK AND THE 103RD ANNUAL MEETING OF THE AMERICAN GASTROENTEROLOGICAL ASSOCIATION; SAN FRANCISCO, CA, USA; MAY 19-22, 2002 ISSN: 0016-5085
- AJUEBOR M ET AL: "CCL3/MIP-1 alpha, a crucial chemokine in the pathogenesis of T cell-mediated hepatitis in mice" BIOSIS, March 2003 (2003-03), XP002263040
- PROUDFOOT A E I ET AL: "STRATEGIES FOR CHEMOKINE ANTAGONISTS AS THERAPEUTICS" SEMINARS IN IMMUNOLOGY, W.B. SAUNDERS COMPANY, PA, US, vol. 15, no. 1, 2003, pages 57-65, XP001156225 ISSN: 1044-5323

### SEQUENCE LISTING

<110> Applied Research Systems ARS Holding N.V.
<120> CC-CHEMOKINE MUTANTS AGAINST LIVER DISEASES
<130> WO801
<160> 3
<170> Patentin version 3.1
<210> 1
   <211> 68
   <212> PRT
   <213> synthetic construct
   <220> Triple 40's RANTES mutant
   <221> source
   <223>
<400> 1
   Ser Pro Tyr Ser Ser Asp Thr Thr Pro Cys Cys Phe Ala Tyr Ile Ala
   1 5 10 15
   Arg Pro Leu Pro Arg Ala His Ile Lys Glu Tyr Phe Tyr Thr Ser Gly
   20 25 30
   Lys Cys Ser Asn Pro Ala Val Val Phe Val Thr Ala Ala Asn Ala Gln
   35 40 45
   Val Cys Ala Asn Pro Glu Lys Lys Trp Val Arg Glu Tyr Ile Asn Ser
   50 55 60
   Leu Glu Met Ser
   65
<210> 2
   <211> 70
   <212> PRT
   <213> synthetic construct
   <220> Triple MIP-1alpha mutant
   <221> source
   <223>
<400> 2
   Ala Ser Leu Ala Ala Asp Thr Pro Thr Ala Cys Cys Phe Ser Tyr Thr
   1 5 10 15
   Ser Ala Gln Ile Pro Gln Asn Phe Ile Ala Asp Tyr Phe Glu Thr Ser
   20 25 30
   Ser Gln Cys Ser Lys Pro Gly Val Ile Phe Leu Thr Lys Ala Ser Ala
   35 40 45
   Gln Val Cys Ala Asp Pro Ser Glu Glu Trp Val Gln Lys Tyr Val Ser
   50 55 60
   Asp Leu Glu Leu Ser Ala
   65 70
<210> 3
   <211> 69
   <212> PRT
   <213> synthetic construct
   <220> Triple MIP-1beta mutant
   <221> source
   <223>
<400> 3
   Ala Pro Met Gly Ser Asp Pro Pro Thr Ala Cys Cys Phe Ser Tyr Thr
   1 5 10 15
   Ala Arg Lys Leu Pro Arg Asn Phe Val Val Asp Tyr Tyr Glu Thr Ser
   20 25 30
   Ser Leu Cys Ser Gln Pro Ala Val Val Phe Gln Thr Ala Ala Ser Ala
   35 40 45
   Gln Val Cys Ala Asp Pro Ser Glu Ser Trp Val Gln Glu Tyr Val Tyr
   50 55 60
   Asp Leu Glu Leu Asn
   65

## Description

### FIELD OF THE INVENTION

The present invention relates to novel therapeutic applications of CC-chemokine mutants having a reduced GAG-binding activity.

### BACKGROUND OF THE INVENTION

Chemokines are secreted pro-inflammatory proteins of small dimensions (70-130 amino acids) mostly involved in the directional migration and activation of cells, especially the extravasation of leukocytes from the blood to tissue localizations needing the recruitment of these cells (Baggiolini M et al., 1997; Rossi D and Zlotnik A, 2000; Fernandez EJ and Lolis E, 2002). Usually chemokines are produced at the site of an injury, inflammation, or other tissue alteration in a paracrine or autocrine fashion, triggering cell-type specific migration and activation.

Depending on the number and the position of the conserved cysteines in the sequence, chemokines are classified into C-, CC-, CXC- and CX₃C-chemokines. Inside each of these families, chemokines can be further grouped according to the homology of the entire sequence, or of specific segments.

A series of heptahelical G-protein coupled membrane receptors, are the binding partners that allow chemokines to exert their biological activity on the target cells, which present specific combinations of receptors according to their state and/or type. An unified nomenclature for chemokine ligands and receptors, which were originally named by the scientists discovering them in a very heterogeneous manner, has been proposed to associate each of these molecule to a systemic name including a progressive number: CCL1, CCL2, etc. for CC-chemokines; CCR1, CCR2, etc. for CC-chemokines receptors, and so on.

The physiological effects of chemokines result from a complex and integrated system of concurrent interactions. The receptors often have overlapping ligand specificity, so that a single receptor can bind different chemokines, as well a single chemokine can bind different receptors. In particular, N-terminal domain of chemokines is involved in receptor binding and N-terminal processing can either activate chemokines or render chemokines completely inactive.

Amongst all the chemokines characterized so far, CC-chemokines, such as CCL5 (also known as RANTES; Appay V and Rowland-Jones SL, 2001) or CCL3 (also known as MIP-1alpha, US 6,355,476), have been intensively studied to identify therapeutically useful molecules. Variants of CC-chemokines, missing up to nine N-terminal amino acids, have been tested for their activity as inhibitors or antagonists of the naturally occurring forms. These molecules are inactive on monocytes and are useful as receptor antagonists (Gong JH et al., 1996; WO 99/16877). Alternatively, N-terminal extension of the mature CC-chemokine with one Methionine results in almost complete inactivation of the molecule, which also behaves as an antagonist for the authentic one (WO 96117935).

Moreover, in order to perform structure-function analysis of CC-chemokines, variants containing substitutions or chemical modifications in different internal positions, as well as CC-chemokine derived peptides, have been tested for the interactions with receptors or other molecules. Some of these variants have been disclosed as having significatively altered binding properties, and sometimes they are active as CC-chemokine antagonists, having potential therapeutic applications in the treatment of HIV infection and some inflammatory or allergic diseases (WO 99/33989; Nardese V et al., 2001). In particular, the binding determinants and the physiological relevance of the interactions of chemokines, by the means of specifically positioned basic residues, with Glycosaminoglycans (GAGs) has been intensively studied (WO 02/28419; Vives R et al., 2002; McCornack MA et al., 2003; Stringer SE et al., 2002; Fukui S et al., 2002; Laurence JS et al., 2001; Martin L et al., 2001; Koopmann W and Krangel MS, 1997).

Even though there are potential drawbacks in using chemokines as therapeutic agents (tendency to aggregate, promiscuous binding), these molecules offer the possibility for therapeutic intervention in pathological conditions associated to such processes, in particular by inhibiting / antagonizing specific chemokines and their receptors at the scope to preventing the excessive recruitment and activation of cells, in particular leukocytes, for a variety of indications related to inflammatory and autoimmune diseases, cancers, and bacterial or viral infections (Schneider GP et al., 2001, Baggiolini M, 2001; Godessart N and Kunkel SL, 2001).

The possible therapeutic applications of chemokine-related compounds against hepatic diseases have been intensively studied, as recently reviewed (Ajuebor MN et al., 2002; Marra F, 2002, Colletti LM, 1999). In particular, liver specific inflammation is mediated by activated CD4(+) T cells and driven by an upregulation of the hepatic expression of IFNgamma, but the mechanisms governing T cell migration from the blood into tissues during T cell-mediated hepatitis remains incompletely understood, since the endogenous mediators that promote the recruitment of T cells to the liver during T cell-mediated liver diseases have been poorly characterized.

It has been demonstrated that some chemokines are highly expressed and important for the recruitment for liver-infiltrating lymphocytes in hepatitis-related animal models (acetaminophen-induced, Concanavalin A-induced, adenovirus-induced, or hepatitis B virus-specific), suggesting a specific role of these molecules in the development of hepatitis (Bautista AP, 2002; Lalor PF et al., 2002; Hogaboam CM et al., 2000; Kusano F et al., 2000).

Some broad spectrum CC-chemokine antagonists were disclosed in connection to hepatic diseases (WO 00/73327; WO 01/58916; US6495515). CXC chemokines are capable to induce rapid hepatocyte proliferation and liver regeneration after injury (WO 01/10899). CCR1 or MIP-1alpha antagonists can be used for inhibiting graft-related or ischemia/reperfusion-related liver dysfunctions (WO 00/44365; Murai M et al., 1999).

However, prior art fails to describe any therapeutic efficacy of an isolated, specific CC-chemokine mutant generated by the substitution of internal residues, against liver fibrotic inflammatory and/or autoimmune diseases.

### SUMMARY OF THE INVENTION

The present application is directed to the Use of a CC-chemokine mutant having a reduced GAG-binding activity in the preparation of a pharmaceutical composition for the treatment of liver fibrotic inflammatory and/or autoimmune diseases, wherein
the CC-chemokine is CCL5 / RANTES and the mutant is triple 40's RANTES mutant (SEQ ID NO:1).

It has been surprisingly found that a CCL5 / RANTES mutant having re duced GAG-binding properties, resulting from the substitution of specific internal residues, counteracts liver fibrotic injury in a relevant animal model.

These evidences demonstrate the possibility of using this and other mutants of CC-chemokines (such as CCL3 / MIP-1alpha or CCL4 / MIP-1beta) having similar reduced GAG-binding activity in the treatment of liver fibrotic inflammatory and/or autoimmune diseases. Particularly preferred are CCL5 mutants are the GAG-binding defective mutants of CCL5 generated by appropriately mutagenising the GAG-binding domain of CCL5.

Features and advantages of the invention will be apparent from the following detailed description.

### DESCRIPTION OF THE FIGURES

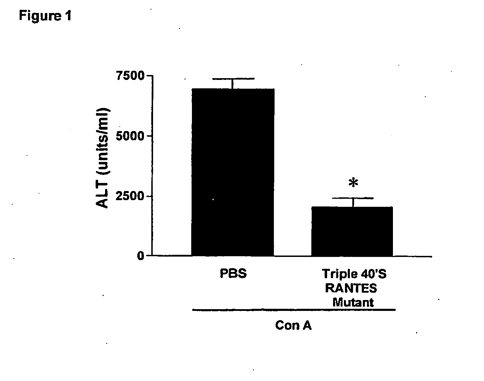

- Figure 1:: effect of the treatment with a GAG-binding defective CCL5 mutant (triple 40's RANTES mutant) in an animal model for liver diseases based on Concanavallin A (Con A) administration and measurement of serum alanine transaminase (ALT). The asterisk indicates the statistical significance of the measured difference (P<0.05) when compared to the PBS-treated controls.

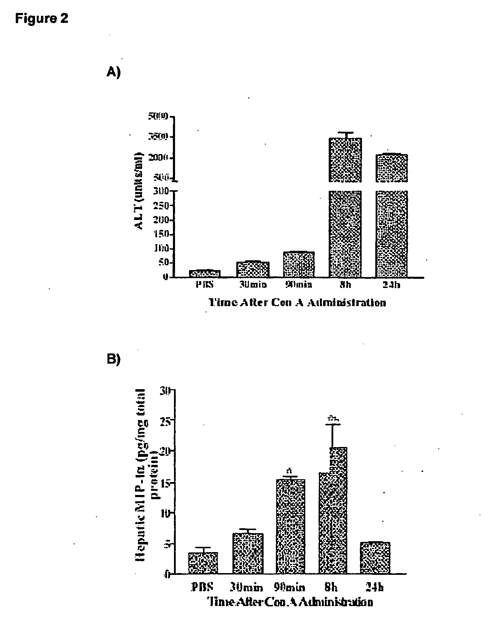

- Figure 2:: time course analysis of the effect of Con A (Con A) administration on serum ALT (A) and on hepatic MIP-1alpha (B) levels in mice.

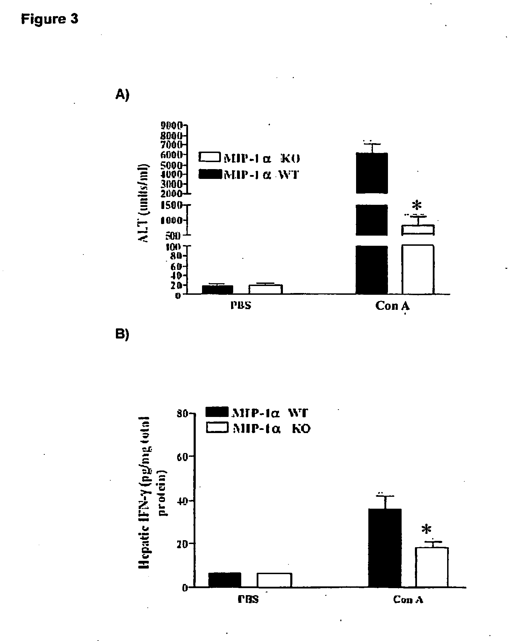

- Figure 3:: effect of the treatment with Con A or PBS on control (MIP-1α WT) and MIP-1alpha knock-out (MIP-1α KO) mice on serum ALT (A) or hepatic IFN-gamma (B) levels in mice. The levels were measured 8 hours after the administration of PBS or Con A. The asterisk indicates the statistical significance of the measured difference (P<0.05) when compared to the PBS-treated controls.

### DETAILED DESCRIPTION OF THE INVENTION

The main object of the present invention is the use of a CC -chemokine mutant having a reduced GAG-binding activity for the treatment of liver fibrotic inflammatory and/or autoimmune diseases. Namely, the Use of a CC-chemokine mutant having a reduced GAG-binding activity in the preparation of a pharmaceutical composition for the treatment of liver fibrotic inflammatory and/or autoimmune diseases, wherein
the CC-chemokine is CCL5 / RANTES and the mutant is triple 40's RANTES mutant (SEQ ID NO:1). CC-chemokine mutants are already disclosed in the prior art for the CC-chemokines CCL3 / MIP-1alpha, CCL4 / MIP-1beta, or CCL5 / RANTES (WO 02/28419; Laurence JS et al., 2001; Koopmann W and Krangel MS. 1997).

In particular, these CC-chemokine mutants have the sequence of the ones disclosed in the prior art under the names of triple 40's RANTES mutant (SEQ ID NO: 1), triple MIP-1alpha mutant (SEQ ID NO: 2), and triple 40's MIP-1beta mutant (SEQ ID NO: 3) mutants. It is however evident that any other corresponding mutant of CCL3 / MIP-1alpha, CCL4 / MIP-1beta, or CCL5 / RANTES having reduced GAG-binding properties resulting from the substitution of the same residues disclosed in the prior art but with a different amino acid (i.e. the basic residue is substituted with a non -polar amino acid other than Ala or with an acid residue), or resulting from a substitution in other position(s) can be used as described herein.

These polypeptides can be prepared by chemical synthesis, by site-directed mutagenesis techniques, or any other known technique suitable thereof, which provide a finite set of substantially corresponding mutated or shortened peptides or polypeptides which can be routinely obtained and tested by one of ordinary skill in the art using the teachings presented in the prior art and in the Examples of the present patent application. Similar compounds may also result from conventional mutagenesis technique of the encoding DNA, from combinatorial technologies at the level of encoding DNA sequence (such as DNA shuffling, phage display/selection), or from computer-aided design studies based on the tridimensional structure and other functional assays of chemokines, with or without the presence of GAGs (Rajarathnam K, 2002; Vives R et al., 2002; McCornack MA et al., 2003; Stringer SE et al., 2003; Fukui S et al., 2002; Martin L et al., 2001).

The above cited prior art on GAG-binding defective CC-chemokine mutants fails to identify liver fibrotic inflammatory and/or autoimmune diseases as therapeutic indications in which these molecules can provide a beneficial effect. As there are currently therapies only partially effective and/or acceptable for treating diseases such as alcoholic liver diseases, viral or autoimmune hepatitis, the disclosed CC-chemokine mutants represent alternative therapeutic compounds possibly better accepted and efficient than the current therapies

The wording "a reduced GAG-binding activity" or "GAG-binding defective" means that the CC-chemokine mutants have a lower ability to bind to GAGs, i. e. a lower percentage of each of these mutants bind to GAGs (like heparin sulphate) with respect to the corresponding wild-type molecule, as measured with the assays in the above cited prior art disclosing such mutants.
In addition to the mutation at the specific positions leading to the decreased affinity for GAGs, the CC-chemokine mutants may include other modifications with respect to the wild-type molecule, generating active variants of said CC-chemokine mutants in which one or more amino acids have been added, deleted, or substituted in a conservative manner. These additional modifications should be intended to maintain, or even improve, the properties of the specific mutants, or by a ny other relevant means known in the art, making them equally useful for treating liver fibrotic inflammatory and/or autoimmune diseases. Other additional preferred changes in these active variants are commonly known as "conservative" or "safe" substitutio ns, that is, with amino acids having sufficiently similar chemical properties, in order to maintain the structure and the biological function of the CC-chemokine mutant. It is clear that insertions and deletions of amino acids may also be made in the above defined sequences without altering their function, particularly if the insertions or deletions only involve a few amino acids, e.g., under ten, and preferably under three, and do not remove or displace amino acids which are critical to the functional conformation of a protein or a peptide.

The literature provide many models on which the selection of conservative amino acids substitutions can be performed on the basis of statistical and physico-chemical studies on the sequence and/or the structure of natural protein (Rogov SI and Nekrasov AN, 2001). Protein design experiments have shown that the use of specific subsets of amino acids can produce foldable and active proteins, helping in the classification of amino acid "synonymous" substitutions which can be more easily accommodated in protein structure, and which can be used to detect functional and structural homologs and paralogs (Murphy LR et al., 2000). The synonymous amino acid groups and more preferred synonymous groups are those defined in Table I.

Alternatively, active CC-chemokine mutants may contain on or more non -natural, amino acid derivatives being "synonymous" to a natural amino acid, are those defined in Table II. By "amino acid derivative" is intended an amino acid or amino acid -like chemical entity other than one of the 20 genetically encoded naturally occurring amino acids. In particular, the amino acid derivative may contain substituted or non-substituted alkyl linear, branched, or cyclic moieties, and may include one or more heteroatoms. The amino acid derivatives can be made de novo or obtained from commercial sources (Calbiochem-Novabiochem AG, Switzerland; Bachem, USA). Various methodologies for incorporating unnatural amino acids derivatives into proteins, using both *in vitro* and *in vivo* translation systems, to probe and/or improve protein structure and function are disclosed in the literature (Dougherty DA, 2000).

The term "active" means that such alternative compounds should maintain the therapeutic properties of the CC-chemokines mutants against liver fibrotic inflammatory and/or autoimmune diseases as described in the present invention, and should be as well pharmaceutically acceptable and useful.

In another embodiment, a polypeptide comprising the GAG-binding defective CC-chemokine mutant and an amino acid sequence belonging to a protein sequence other than the corresponding CC-chemokine can be also used for treating liver fibrotic inflammatory and/or autoimmune diseases. The heterologous sequence is intended provide additional properties without considerably impairing the therapeutic activity. Examples of such additional properties are an easier purification procedure, a longer lasting half-life in body fluids, an additional binding moiety, the maturation by means of an endoproteolytic digestion, or extracellular localization. This latter feature is of particular importance for defining a specific group of fusion or chimeric proteins included in the above definition since it allows the CC-chemokine mutants to be localized in the space where not only where the isolation and purification of these polypeptides is facilitated, but also where CC-chemokines naturally interact with receptors and other molecules. Design of the moieties, ligands, and linkers, as well methods and strategies for the construction, purification, detection and use of fusion proteins are widely discussed in the literature (Nilsson J et al., 1997; "Applications of chimeric genes and hybrid proteins" Methods Enzymol. Vol. 326-328, Academic Press, 2000; WO 01/77137).

Additional protein sequences can be chosen amongst extracellular domains of membrane-bound protein, immunoglobulin constant regions, multimerization domains, extracellular proteins, signal peptide-containing proteins, export signal-containing proteins. The choice of one or more of these sequences to be fused to the CC-chemokine mutants is functional to the desired use, delivery and/or preparation method.

The GAG-binding defective CC-chemokine mutants can be also provided for the treatment of liver fibrotic inflammatory and/or autoimmune diseases in the form of the corresponding active precursor, salt, derivative, conjugate or complex. These alternative forms may be preferred according to the.desired method of delivery and/or production.

The "precursors" are compounds which can be converted into other compounds by metabolic and enzymatic processing prior or after the administration to the cells or to the organism.

The term "salts" herein refers to both salts of carboxyl groups and to acid addition salts of amino groups of the peptides, polypeptides, or analogs thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid. Any of such salts should have substantially similar activity to the original polypeptide.

The term "fractions" as herein used refers to derivatives which can be prepared from the functional groups present on the lateral chains of the amino acid moieties or on the N-/ or C-terminal groups according to known methods. Such derivatives include for example esters or aliphatic amides of the carboxyl-groups and N-acyl derivatives of free amino groups or O-acyl derivatives of free hydroxyl-groups and are formed with acyl-groups as for example alcanoyl - or aroyl-groups. Alternatively, the derivatives may contain sugars or phosphates groups linked to the functional groups present on the lateral chains of the amino acid moieties. Such molecules can result from *in vivo* or *in vitro* processes which do not normally alter primary sequence, for example chemical derivativization of peptides (acetylation or carboxylation), phosphorylation (introduction of phosphotyrosine, phosphoserine, or phosphothreonine residues) or glycosylation (by exposing the peptide to enzymes which affect glycosylation e.g., mammalian glycosylating or deglycosylating enzymes).

The term "derivatives" as herein used refers to derivatives which can be prepared from the functional groups present on the lateral chains of the amino acid moieties or on the N- or C-terminal groups according to known methods. Such derivatives include for example esters or aliphatic amides of the carboxyl-groups and N-acyl derivatives of free amino groups or O-acyl derivatives of free hydroxyl-groups and are formed with acyl-groups as for example alcanoyl- or aroyl-groups.

Alternatively, useful conjugates or complexes of the CC-chemokine mutants can be generated by using molecules and methods known in the art for improving the detection of the interaction with other proteins (radioactive or fluorescent labels, biotin), therapeutic efficacy (cytotoxic agents, isotopes), or drug delivery efficacy, such as polyethylene glycol and other natural or synthetic polymers (Pillai O and Panchagnula R, 2001). In the latter case, a site-directed modification of an appropriate residue, present in the natural sequence or introduced by mutating the natural sequence, at an internal or terminal position, can be introduced. Similar modifications have been already disclosed for chemokines (WO 02/04499; WO 02/04015; Vita C et al., 2002).

Any residue can be used for attachment, provided it has a side-chain amenable for polymer attachment (i.e., the side chain of an amino acid bearing a functional group, e.g., lysine, aspartic acid, glutamic acid, cysteine, histidine, etc.). Alternatively, a residue at these sites can be replaced with a different amino acid having a side chain amenable for polymer attachment. Polymers suitable for these purposes are biocompatible, namely, they are non-toxic to biological systems, and many such polymers are known. Such polymers may be hydrophobic or hydrophilic in nature, biodegradable, non-biodegradable, or a combination thereof. These polymers include natural polymers (such as collagen, gelatin, cellulose, hyaluronic acid), as well as synthetic polymers (such as polyesters, polyorthoesters, polyanhydrides). Examples of hydrophobic non-degradable polymers include polydimethyl siloxanes, polyurethanes, polytetrafluoroethylenes, polyethylenes, polyvinyl chlorides, and polymethyl methaerylates. Examples of hydrophilic non-degradable polymers include poly(2-hydroxyethyl methacrylate), polyvinyl alcohol, poly(N-vinyl pyrrolidone), polyalkylenes, polyacrylamide, and copolymers thereof. Preferred polymers comprise as a sequential repeat unit ethylene oxide, such as polyethylene glycol (PEG).

The preferred method of attachment employs a combination of peptide synthesis and chemical ligation. Advantageously, the attachment of a water-soluble polymer will be through a biodegradable linker, especially at the N-terminal region of a protein. Such modification acts to provide the protein in a "pro-drug" form that, upon degradation of the linker, releases the protein without polymer modification.

The GAG-binding defective CC-chemokine mutants may be prepared by any appropriate procedure in the art, such as recombinant DNA -related technologies involving the expression in Eukaryotic cells (e.g. yeasts, insect or mammalian cells) or Prokaryotic cells. Detailed methods for producing the GAG-binding defective CC-chemokine mutants can be found in the prior art originally disclosing them (WO 02/28419; Laurence JS et al., 2001; Koopmann W and Krangel MS, 1997), as well as in other literature featuring protocols for chemokine production (Edgerton MD et al., 2000) or common molecular biology techniques for the production of recombinant proteins in Prokaryotic or Eukaryotic host cells, such as some titles in the series "A Practical Approach" published by Oxford University Press ("DNA Cloning 2: Expression Systems", 1995; "DNA Cloning 4: Mammalian Systems", 1996; "Protein Expression", 1999; "Protein Purification Techniques", 2001).

Alternatively the GAG-binding defective CC-chemokine mutants may be prepared by any other well known procedure in the art, in particular, by the well established chemical synthesis procedures, which can be efficiently applied on these mol ecule given the short length. Totally synthetic chemokines, also containing additional chemical groups, are disclosed in the literature (Brown A et al., 1996; Vita C et al., 2002).

Examples of chemical synthesis technologies are solid phase synthesis and liquid phase synthesis. As a solid phase synthesis, for example, the amino acid corresponding to the N-terminus of the peptide to be synthetized is bound to a support which is insoluble in organic solvents, and by alternate repetition of reactions, one wherein amino acids with their amino groups and side chain functional groups protected with appropriate protective groups are condensed one by one in order from the C-terminus to the N-terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CI-Z (2-chlorobenzyloxycarbonyl), Br-Z (2-bromobenzyloxycarbonyl), Bzl (benzyl), Fmoc (9-fluorenylmethoxycarbonyl), Mbh (4,4'-dimethoxydibenzhydryl). Mtr (4-methoxy-2,3,6-trimethylbenzenesulphonyl), Trt (trityl), Tos (tosyl), Z (benzyloxycarbonyl) and Cl2-Bzl (2,6-dichlorobenzyl) for the amino groups; NO2 (nitro) and Pmc (2,2,5,7,8-pentamethylchromane-6-sulphonyl) for the guanidino groups); and tBu (t-butyl) for the hydroxyl groups). After the synthesis, the desired peptide is subjected to the de-protection reaction and cut out from the solid support. Such peptide cutting reaction may be carried with hydrogen fluoride or trifluoromethane sulfonic acid for the Boc method, and with TFA for the Fmoc method. Finally, the intact full-length peptides are purified and chemically or enzymatically folded (including the formation of disulphide bridges between cysteines) into the corresponding CC-chemokine mutants.

Purification of the natural, synthetic or recombinant proteins is carried out by any one of the methods known for this purpose, i.e. any conventional procedure involving extraction, precipitation, chromatography, electrophoresis, or the like. A further purification procedure that may be used in preference for purifying the protein is affinity chromatography using monoclonal antibodies, heparin, or any other suitable ligand that can bind the target protein at high efficiency and can be immobilized on a gel matrix contained within a column. Impure preparations containing the proteins are passed through the column. The protein will be bound to the column by means of this ligand while the impurities will pass through. After washing, the protein is eluted from the gel by a change in pH or ionic strength. Alternatively, HPLC (High Performance Liquid Chromatography) can be also used.

Described herein is the use of a CC-chemokine mutant, wherein the CC-chemokine is CCL3 / MIP-1alpha, CCL4 / MIP-1beta, or CCL5 / RANTES, having reduced GAG-binding activity in the preparation of a pharmaceutical composition for liver inflammatory and/or fibrotic diseases, in particular when formulated in combination with pharmaceutically acceptable carriers, excipients, stabilizers, adjuvants, or diluents.

A non-limitative list of disorders involving hepatic damage in which the CC-chemokine mutant having reduced GAG-binding activity can be used includes alcoholic liver diseases (cirrohosis, steatosis), a viral hepatitis, an autoimmune hepatitis, or any other liver fibrotic degeneration.

The CC-chemokine mutants may be used alone, or with another therapeutic composition acting synergically or in a coordinated / sequential ma nner with them in the treatment of liver inflammatory and/or fibrotic diseases. For example, similar synergistic properties of CC-chemokine mutants have been demonstrated in combination with cyclosporin (WO 00/16796).

In view of the claimed uses and methods of treatment, any drug delivery method allowing the targeting of the GAG-binding defective CC-chemokine mutant is preferred. Similar methods are known in the prior and may involve the conjugation of the CC-chemokine mutant with galactosylated or mannosyl ated albumin (Chuang VT et al., 2002) or the synthesis of polymeric nanoparticles from a sugar-containing conjugate composed of lactobionic acid, diamine-terminated polyethylene glycol) and cholic acid (Kim IS and Kim SH, 2002).

An "effective amount" refers to an amount of the active ingredients that is sufficient to affect the course and the severity of the disease, leading to the reduction or remission of the liver pathology. The effective amount will depend on the route of administration and the condition of the patient.

The pharmaceutical compositions may be formulated in any acceptable way to meet the needs of the mode of administration for treating liver diseases. For example, the use of biomaterials and other polymers for drug delivery, as well the different techniques and models to validate a specific mode of administration, are disclosed in literature (Luo B and Prestwich GD, 2001; Cleland JL et al., 2001).

"Pharmaceutically acceptable" is meant to encompass any carrier, which does not interfere with the effectiveness of the biological activity of the active ingredient and that is not toxic to the host to which is administered. Carriers can be selected also from starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the various oils, including those of petroleum, animal, vegetable or synthetic origin (peanut oil, soy bean oil, mineral oil, sesame oil). For example, for parenteral administration, the above active ingredients may be formulated in unit dosage form for injection in vehicles such as saline, dextrose solution, serum albumin and Ringer's solution.

Besides the pharmaceutically acceptable carrier, the compositions of the invention can also comprise minor amounts of additives, such as stabilizers, excipients, buffers and preservatives that may facilitate the processing of the active compounds into preparations which can be used pharmaceutically. Moreover, these compositions may contain another active ingredient that can act synergically or in a coordinated manner with the CC-chemokine mutants.

The administration of such active ingredient may be by intravenous, intramuscular or subcutaneous route. Other routes of administration, which may establish the desired effects of the respective ingredients in the liver, are comprised by the present invention. For example, administration may be by various parenteral routes such as subcutaneous, intravenous, intradermal, intramuscular, intraperitoneal, intranasal, transdermal, oral, or buccal routes. The pharmaceutical compositions of the present invention can also be administered in sustained or controlled release dosage forms, including depot injections, osmotic pumps, and the like, for the prolonged administration of the polypeptide at a predetermined rate, preferably in unit dosage forms suitable for single administration of precise dosages.

Parenteral administration can be by bolus injection or by gradual perfusion over time. Preparations for parenteral administration include sterile aqueous or non-aqueous solutions, suspensions, and emulsions, which may contain auxiliary agents or excipients known in the art, and can be prepared according to routine methods. In addition, suspension of the active compounds as appropriate oily injection suspensions may be administered. Suitable lipophilic solvents or vehicles include fatty oils, for example, sesame oil, or synthetic fatty acid esters, for example, sesame oil, or synthetic fatty acid esters, for example, ethyl oleate or triglycerides. Aqueous injection suspensions that may contain substances increasing the viscosity of the suspension include, for example, sodium carboxymethyl cellulose, sorbitol, and/or dextran. Optionally, the suspension may also contain stabilizers. Pharmaceutical compositions include suitable solutions for administration by injection, and contain from about 0.01 to 99.99 percent, preferably from about 20 to 75 percent of active compound together with the excipient.

The optimal dose of active ingredient may be appropriately selected according to the route of administration, patient conditions and characteristics (sex, age, body weight, health, size), extent of symptoms, concurrent treatments, frequency of treatment and the effect desired. Adjustment and manipulation of established dosage ranges are well within the ability of those skilled.

Usually a daily dosage of active ingredient can be about 0.01 to 100 milligrams per kilogram of body weight. Ordinarily 1 to 40 milligrams per kilogram per day given in divided doses or in sustained release form is effective to obtain the desired results. Second or subsequent administrations can be performed at a dosage, which is the same, less than, or greater than the initial or previous dose administered to the individual.

The present invention has been described with reference to the specific embodiments, but the content of the description comprises all modifications and substitutions, that can be brought by a person skilled in the art without extending beyond the meaning and purpose of the claims.

The invention will now be described by means of the following Examples, which should not be construed as in any way limiting the present invention. The Examples will refer to the Figures specified here below.

### EXAMPLES

### Example 1: efficacy of different CCL5 mutant having a reduced GAG-binding activity in a hepatitis model

Concanavalin A (Con A)-induced liver injury depends on the activation of recruitment of CD4(+) T cells by macrophages. In general, T cells are the driving force underlying immunologically mediated hepatic disorders, making therefore the Con -A model highly relevant for studying the pathophysiology of diseases such as autoimmune or acute hepatitis (Takeda K et al., 2000; Tiegs G et al., 1992).

The assay involves the measurement of serum alanine aminotransferase (ALT), a liver enzyme contained in hepatocytes and released into serum when these cells are damaged. ALT is the most widely used marker in humans and animals to document damage and destruction of liver cells (as in hepatitis), and, generally, ALT concentration correlates with histological changes.

As example of CC-chemokine mutant having a reduced GAG-binding activity, the CCL5 mutant called triple 40's RANTES mutant was chosen and expressed in *E*. *coli* and purified as previously described (WO 02/28419).

Specific pathogen-free male C57BU6 mice (body weight of 21-23 grams; Charles River Breeding Farms) were treated with Phosphate Buffer Saline (PBS; vehicle control; 0.1 ml), or with one of above described CCL5 mutants (30 micrograms/mouse in 0.1 ml PBS). The mice (5-10 per group) were injected s.c. 1 hour prior to Con-A i.v. injection (freshly prepared Con A type V, 0.25 mg/mouse in 0.1 ml PBS; Sigma). At 8 hours after Con A administration and under halothane anaesthesia, blood was collected for measuring plasma ALT using a commercial kit for ALT determination (Sigma).

This biochemical approach for quantifying liver injury showed that the triple 40's RANTES mutant is capable of reducing ALT concentration in serum of the pre-treated animals in a statistically significant manner (figure 1).

### Example 2: relevance of MIP-1alpha expression in the Concanavallin A mouse models (not part of the invention)

A time course experiments on normal mice was performed to verify if there is any correlation between ALT levels and hepatic CCL3 / MIP-1alpha protein concentration in control male C57BL/6J mice and CCL3 / MIP-1alpha knock-out C57BL/6J (Cook DN et al., 1995).

Mice (body weight of 21-23 grams; 5-6 weeks old; commercially available through Jackson Laboratories and Charles River Breeding Farms) were injected intravenously with freshly prepared Concanavallin A (Con A; 13.5 mg/kg; Sigma) in 0.1 ml Phosphate Buffer Saline (PBS), or with 0.1 PBS only (vehicle control).

The levels of serum alanine transaminase (ALT) were measured using a commercial kit (Sigma). Blood was collected from mice at 30 minutes, 9 0 minutes, 8 hours and 24 hours after Con A administration and under halothane anesthesia.

In a separate set of experiments, the levels of hepatic CCL3/MIP-1alpha or IFNgamma were determined in control and CCL3/MIP-1alpha knock-out mice that were injected with Con A or PBS only. Mice were sacrificed at th e indicated time points after Con A or PBS injection and livers were perfused with ice-cold sterile PBS to remove blood elements. Individual perfused livers were homogenised in ice -cold PBS buffer containing protease cocktail inhibitor (Sigma) immediately after their removal, tissue homogenates were centrifuged twice, and the supernatants filtered through a 0.45 -mm filter and stored at -80°C until used for protein determination. Hepatic CCL3 / MIP-1alpha and IFNgamma levels were measured by specific murine ELISA following a published protocol (Bone-Larson CL et al., 2001), and using commercial antibodies (R&D Systems). Total protein concentration in the homogenates was calculated using a commercial protein colorimetric assay (Bio-Rad Laboratories).

The data demonstrated that maximum hepatic injury, as evaluated by using ALT concentration, and peak hepatic CCL3 / MIP-1alpha levels can be both observed at 8 hours after intravenous administration of a single dose of Con A (13.5 mg/kg). This compound caused a more than 100-fold increase of ALT levels (figure 2A) paralleled by a more than five-fold increase of hepatic CCL3 / MIP-1alpha protein expression (figure 2B) above control PBS injection.

The effect of Con A administration in transgenic mice lacking the CCL3/ MIP-1alpha gene to verify if CCL3 / MIP-1alpha deficiency impairs the development of Con A-induced hepatic injury. In fact, CCL3 / MIP-1alpha knock-out mice exhibited significantly less hepatic injury 8 hours after the Con A injection relative to control m ice, as demonstrated biochemically by a significant reduction (approx. five -fold) in ALT level (figure 3A). Moreover, hepatic concentration of IFNgamma, one of the cytokines implicated in the pathogenesis of Con A-induced hepatitis since it is produced by CD4(+) T cells recruited to the liver (Mizuhara H et al., 1996), is significantly lowered in

CCL3 / MIP-1alpha knock-out mice at 8 hours after Con A administration, when compared to control mice (figure 3B).

All data are shown as Mean ± Standard Deviation. For comparisons of means between 2 experimental groups (n=4-10 mice per group) a Student's unpaired t-test was used, considering P value <0.05 statistically significant. Statistical analyses were performed using GraphPad Instat (version 3.00) Software.

### Conclusions

The analysis of the data generated with the substituted CCL5 mutant demonstrate how, using a single CC-chemokine mutant having the specific binding profile characterized in the prior art (WO 02/28419; Laurence JS et al., 2001; Koopmann W and Krangel MS, 1997), a surprising therapeutically relevant effect can be obtained, suggesting the use of similar molecules in the treatment of fibrotic liver diseases involving inflammatory and/or autoimmunitary reactions. The comparison with results obtained using other animal models for such diseases (acetaminophen-induced or adenovirus-induced hepatitis), may provide further confirmation of the therapeutic applicability of this specific CC-chemokine mutant.

**TABLE I**

| **Amino Acid** | **Synonymous Group** | **More Preferred Synonymous Groups** |
|---|---|---|
| **Ser** | Gly, Ala, Ser, Thr, Pro | Thr, Ser |
| **Arg** | Asn, Lys, Gln, Arg, His | Arg, Lys, His |
| **Leu** | Phe, Ile, Val, Leu, Met | Ile, Val, Leu, Met |
| **Pro** | Gly, Ala, Ser, Thr, Pro | Pro |
| **Thr** | Gly, Ala, Ser, Thr, Pro | Thr, Ser |
| **Ala** | Gly, Thr, Pro, Ala, Ser | Gly, Ala |
| **Val** | Met, Phe, Ile, Leu, Val | Met, Ile, Val, Leu |
| **Gly** | Ala, Thr, Pro, Ser, Gly | Gly, Ala |
| **Ile** | Phe, Ile, Val, Leu, Met | Ile, Val, Leu, Met |
| **Phe** | Trp, Phe,Tyr | Tyr, Phe |
| **Tyr** | Trp, Phe,Tyr | Phe, Tyr |
| **Cys** | Ser, Thr, Cys | Cys |
| **His** | Asn, Lys, Gln, Arg, His | Arg, Lys, His |
| **Gln** | Glu, Asn, Asp, Gln | Asn, Gln |
| **Asn** | Glu, Asn, Asp, Gln | Asn, Gln |
| **Lys** | Asn, Lys, Gln, Arg, His | Arg, Lys, His |
| **Asp** | Glu, Asn, Asp, Gln | Asp, Glu |
| **Glu** | Glu, Asn, Asp, Gln | Asp, Glu |
| **Met** | Phe, Ile, Val, Leu, Met | Ile, Val, Leu, Met |
| **Trp** | Trp, Phe,Tyr | Trp |

**TABLE II**

| **Amino Acid** | **Synonymous Group** |
|---|---|
| Ser | D-Ser, Thr, D-Thr, allo-Thr, Met, D-Met, Met(O), D-Met(O), L-Cys, D-Cys |
| Arg | D-Arg, Lys, D-Lys, homo-Arg, D-homo-Arg, Met, Ile, D-.Met, D-ile, Orn, D-Om |
| Leu | D-Leu, Val, D-Val, AdaA, AdaG, Leu, D-Leu, Met, D-Met |
| Pro | D-Pro, L-I-thioazolidine-4-carboxylic acid, D-or L-1-oxazolidine-4-carboxylic acid |
| Thr | D-Thr, Ser, D-Ser, allo-Thr, Met,D-Met, Met(O), D-Met(O), Val, D-Val |
| Ala | D-Ala, Gly, Aib, B-Ala, Acp, L-Cys, D-Cys |
| Val | D-Val, Leu, D-Leu, Ile, D-Ile, Met, D-Met, AdaA, AdaG |
| Gly | Ala, D-Ala, Pro, D-Pro, Aib, beta.-Ala, Acp |
| Ile | D-Ile, Val, D-Val, AdaA, AdaG, Leu, D-Leu, Met, D-Met |
| Phe | D-Phe, Tyr, D-Thr, L-Dopa, His, D-His, Trp, D-Trp, Trans-3,4, or 5-phenylproline, AdaA, AdaG, cis-3,4, or 5-phenylproline, Bpa, D-Bpa |
| Tyr | D-Tyr, Phe, D-Phe, L-Dopa, His, D-His |
| Cys | D-Cys, S-Me-Cys, Met, D-Met, Thr, D-Thr |
| Gln | D-Gln, Asn, D-Asn, Glu, D-Glu, Asp, D-Asp |
| Asn | D-Asn, Asp, D-Asp, Glu, D-Glu, Gln, D-Gln |
| Lys | D-Lys, Arg, D-Arg, homo-Arg, D-homo-Arg, Met, D-Met, Ile, D-Ile, Orn, D-Om |
| Asp | D-Asp, D-Asn, Asn, Glu, D-Glu, Gln, D-Gln |
| Glu | D-Glu, D-Asp, Asp, Asn, D-Asn, Gln, D-Gln. |
| Met | D-Met, S-Me-Cys, Ile, D-Ile, Leu, D-Leu, Val, D-Val |

### REFERENCES

Ajuebor MN et al., Biochem Pharmacol 2002, 63:1191-6.
Appay V and Rowland-Jones SL, Trends Immunol 2001, 22: 83 -7.
Baggiolini M et al., Annu Rev Immunol 1997, 15: 675-705.
Baggiolini M, J Intern Med 2001, 250: 91-104.
Bone-Larson CL et al., J Immunol 2001, 167: 7077-83.
Brown A et al., J Pept Sci 1996, 2: 40-6.
Bautista AP, Front Biosci 2002, 7: a117-25.
Chuang VT et al., Pharm Res 2002, 19: 569 -77.
Cleland JL et al., Curr Opin Biotechnol 2001,12: 212-9.
Colletti LM, Drug News Perspect 1999, 12: 5-11.
Cook DN et al., Science 1995, 269:1583-1585.
Dougherty DA, Curr Opin Chem Biol 2000, 4: 645-52.
Edgerton MD et al., Methods Mol Biol 2000, 138: 33-40.
Fernandez EJ and Lolis E, Annu Rev Pharmacol Toxicol 2002, 42:469-499.
Fukui S et al., Nat Biotechnol 2002, 20: 1011-7.
Godessart N and Kunkel SL, Curr Opin Immunol 2001, 13: 670-675.
Gong JH et al., J Biol Chem 1996, 271: 10521-7.
Hogaboam CM et al., Am J Pathol 2000, 156: 1245-52.
Kim IS and Kim SH, Int J Pharm 2002, 245: 67-73.
Koopmann Wand Krangel MS, J Biol Chem 1997, 272: 10103-9.
Kusano F et al., Lab Invest. 2000, 80: 415-22.
Lalor PF et al., Immunol Cell Biol 2002, 80: 52 -64.
Laurence JS et al., Biochemistry 2001, 40: 4990-9.
Luo B and Prestwich GD, Exp Opin Ther Patents 2001, 11: 1395-1410.
Marra F, Front Biosci 2002, 7: d1899-914.
Martin L et al., Biochemistry 2001, 40: 6303-18.
McCornack MA et al., J Biol Chem 2003, 278:1946-56 (epub ahead of print on Oct. 30th 2002).
Mizuhara H et al., Hepatology 1996, 23: 1608-1615.
Murai M et al., J Clin Invest 1999, 104: 49-57.
Murphy LR et al., Protein Eng 2000, 13:149-52.
Nardese V et al., Nat Struct Biol 2001, 8: 611-5.
Nilsson J et al., Protein Expr Purif 1997, 11: 1-16.
Pillai O and Panchagnula R, Cur Opin Chem Biol 2001, 5: 447 -451.
Rajarathnam K, Curr Pharm Des 2002, 8: 2159-69.
Rogov SI and Nekrasov AN, Protein Eng, 14: 459-463, 2001.
Rossi D and Zlotnik A, Annu Rev Immunol 2000, 18:217-42.
Schneider GP et al., Int J Mol Med 2001, 8: 235-44.
Stringer SE et al., Blood 2003, 101: 2243-5 (epub ahead of print on Oct. 24th 2002).
Takeda K et al., Proc Natl Acad Sci U S A 2000, 97: 5498-503.
Tiegs G et al., J Clin Invest 1992, 90: 196-203.
Vita C et al., J Immunol Methods 2002, 266: 53-65.
Vives R et al., Biochemistry 2002, 41: 14779-89.

## Claims

1. Use of a CC-chemokine mutant having a reduced GAG-binding activity in the preparation of a pharmaceutical composition for the treatment of liver fibrotic inflammatory and/or autoimmune diseases, wherein
the CC-chemokine is CCL5 / RANTES and the mutant is triple 40's RANTES mutant (SEQ ID NO:1).

2. The use of claim 1 wherein the CC-chemokine mutant is an active variant of said CC-chemokine mutant in which one or more amino acids have been inserted, deleted, or substituted in a conservative manner.

3. The use of claim 1 or 2 wherein the CC-chemokine mutant is comprised in a polypeptide additionally comprising an amino acid sequence belonging to a protein sequence other than the corresponding CC-chemokine.

4. The use of any of the claims from 1 to 3 wherein the CC-chemokine mutant is in the form of an active precursor, salt, derivative, conjugate or complex.

5. The use of any of the preceding claims wherein the liver disease is an alcoholic liver disease, a viral hepatitis, or an autoimmune hepatitis

## Patentansprüche

1. Verwendung einer CC-Chemokin-Mutante mit einer verringerten GAG-Bindungsaktivität zur Herstellung einer pharmazeutischen Zusammensetzung zur Behandlung von Autoimmunerkrankungen und/oder fibrotischen entzündlichen Erkrankungen der Leber, wobei es sich bei dem CC-Chemokin um CCL5/RANTES und bei der Mutante um die 40er-RANTES-Dreifachmutante (SEQ ID NO: 1) handelt.

2. Verwendung nach Anspruch 1, wobei die CC-Chemokin-Mutante eine aktive Variante der CC-Chemokin-Mutante ist, bei der eine oder mehrere Aminosäuren eingefügt, deletiert oder auf konservative Weise substituiert worden sind.

3. Verwendung nach Anspruch 1 oder 2, wobei die CC-Chemokin-Mutante in einem Polypeptid enthalten ist, das zusätzlich eine Aminosäuresequenz enthält, die zu einer anderen Proteinsequenz als der des entsprechenden CC-Chemokins gehört.

4. Verwendung nach einem der Ansprüche 1 bis 3, wobei die CC-Chemokin-Mutante in Form eines aktiven Vorläufers, Salzes, Derivats, Konjugats oder Komplexes vorliegt.

5. Verwendung nach einem der vorhergehenden Ansprüche, wobei es sich bei der Lebererkrankung um eine alkoholische Lebererkrankung, eine Virushepatitis oder eine Autoimmunhepatitis handelt.

## Revendications

1. Utilisation d'un mutant de chimiokine CC ayant une activité de fixation aux GAG réduite pour la préparation d'une composition pharmaceutique pour le traitement des maladies inflammatoires ou auto-immunes hépatiques, fibrotiques, dans lequel :
la chimiokine CC est CCL5/RANTES et le mutant est le triple mutant RANTES 40's (SEQ ID NO : 1).

2. Utilisation selon la revendication 1, dans laquelle le mutant de chimiokine CC est un variant actif dudit mutant de chimiokine CC dans lequel un ou plusieurs acides aminés ont été insérés, délétés ou substitués de manière conservatrice.

3. Utilisation selon la revendication 1 ou 2, dans laquelle le mutant de chimiokine CC est compris dans un polypeptide comprenant de plus une séquence en acides aminés appartenant à une séquence protéique différente de celle de la chimiokine CC correspondante.

4. Utilisation selon l'une quelconque des revendications 1 à 3, dans laquelle le mutant de chimiokine CC est sous la forme d'un précurseur actif, d'un sel, d'un dérivé, d'un conjugué ou d'un complexe.

5. Utilisation selon l'une quelconque des revendications précédentes, dans laquelle la maladie du foie est une maladie du foie alcoolique, une hépatite virale, ou une hépatite auto-immune.
